# EUROPEAN PATENT APPLICATION

(11) **EP 1 779 781 A2**
(43) Date of publication of application: **02.05.2007**
(21) Application number: 06255534.7
(22) Date of filing: 27.10.2006
(51) Int. Cl.: A61B 5/151, A61B 10/00

(54) **Analyte monitoring system with integrated lancing apparatus**

(30) Priority: 28.10.2005 US 262205
(71) Applicant: Lifescan Scotland Ltd, Inverness-shire IV2 3ED (GB)
(72) Inventor: Sansom, Gordon George, Inverness Inverness-shire IV2 6DN (GB)
(74) Representative: Tunstall, Christopher Stephen

(57) **Abstract**

An analyte monitoring system for the determination of an analyte (such as glucose) in a bodily fluid sample (e.g., blood) includes an external system housing, a lancing apparatus and a meter for determination of the analyte. The lancing apparatus is integrated with the external system housing and includes an inner housing, a firing mechanism, a lancing mechanism and a linkage arm. The firing mechanism is configured for producing a firing force in a first direction. The lancing mechanism is configured for delivering a lancing force in a second direction with the second direction being toward a target site and in opposition to the first direction. The linkage arm is pivotably attached to the housing and has first and second ends engaged to the firing and lancing mechanisms, respectively. During use, pivoting of the linkage arm converts the firing force in the first direction into the lancing force in the opposing second direction.

## Description

### BACKGROUND OF THE INVENTION

1. Field of the Invention

The present invention relates, in general, to medical devices and systems and, in particular, to lancing devices and associated systems.

2. Description of the Related Art

A variety of medical conditions, such as diabetes, call for the monitoring of an analyte concentration (e.g., glucose concentration) in a blood, interstitial fluid or other bodily fluid sample. Typically, such monitoring requires the extraction of a bodily fluid sample from a target site (e.g., a dermal tissue target site on a user's finger). The extraction (also referred to as "expression") of a bodily fluid sample from the target site generally involves lancing the dermal tissue target site with a lancing device and then expressing the bodily fluid sample from the lanced site.

Conventional lancing devices typically have a rigid housing and a lancet that can be armed (also referred to as "primed") and launched (also referred to as "fired") so as to protrude from one end of the lancing device. For example, conventional lancing devices can include a lancet that is mounted within a rigid housing such that the lancet is movable relative to the rigid housing along a longitudinal axis thereof. Typically, the lancet is spring loaded and launched, upon release of the spring, to penetrate (i.e., "lance") a target site (e.g., a dermal tissue target site). A biological fluid sample (e.g., a whole blood sample or interstitial fluid (ISF) sample) can then be expressed from the penetrated target site for collection and analysis. Conventional lancing devices are described in, for example, U.S. Patent No. 5,730,753 to Morita, U.S. Patent No. 6,045,567 to Taylor et al. and U.S. Patent No. 6,071,250 to Douglas et al., each of which is incorporated fully herein by reference.

The lancing of a dermal tissue target site by a conventional lancing device can be unduly painful for several reasons. First, post-launching recoil can cause a lancet to re-penetrate a target site, albeit at a site slightly skewed point with respect to the original lancet penetration point. Such post-launching recoil can, therefore, result in unintentional multiple lancing and an increase in pain. Second, conventional lancing devices may rely on the spring constant of a lancing spring to define a lancet's penetration depth. However, over time the spring constant may change, thus detrimentally altering the penetration depth. Third, a sudden motion-based impulse emanating from the lancing device housing (i.e., a side-effect of launching) may be noticed by a user. The anticipation of such impulses may be disconcerting to the user. Moreover, conventional lancing devices can be large and cumbersome to use.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, in which like numerals represent like elements, of which:

FIG. 1 is a simplified perspective view of a lancing apparatus according to an exemplary embodiment of the present invention;

FIG. 2 is simplified cross-sectional view of the lancing apparatus of FIG. 1;

FIG. 3 is a simplified perspective view of a portion of the lancing apparatus of FIG. 1;

FIG. 4 is a simplified side view of a connector that can be used with embodiments of lancing apparatuses and analyte monitoring systems according the present invention;

FIG. 5 is a simplified side view of the connector of FIG. 4 gripping an integrated medical device;

FIG. 6 is a simplified perspective, cut-away view of the connector and integrated medical device of FIG. 5;

FIGs. 7, 8, 9 and 10 are simplified perspective and cut-away views of the lancing apparatus of FIG. 1 in use, with arrows A, A' and A" indicating movement of a linkage arm of the lancing apparatus;

FIG. 11 is a simplified perspective view of an analyte monitoring system according to an exemplary embodiment of the present invention with a lid of the analyte monitoring system in an open position (i.e., a first position);

FIG. 12 is a simplified perspective and cut-away view of a medical device package containing an integrated medical device as can be employed with embodiments of the present invention;

FIG. 13 is a simplified perspective view of the analyte monitoring system of FIG. 11 depicting the lid in an open position and a medical device package being inserted into a lancing apparatus of the analyte monitoring system;

FIG. 14 is a simplified perspective view representing a portion of FIG. 13;

FIG. 15 is a simplified perspective view of the lid and lancing apparatus of the monitoring system of FIG. 11 depicting the lid in a closed position (i.e., a second position); and

FIG. 16 is a simplified perspective view of the analyte monitoring system of FIG. 11 in use in the hand (H) of a user.

### DETAILED DESCRIPTION OF THE INVENTION

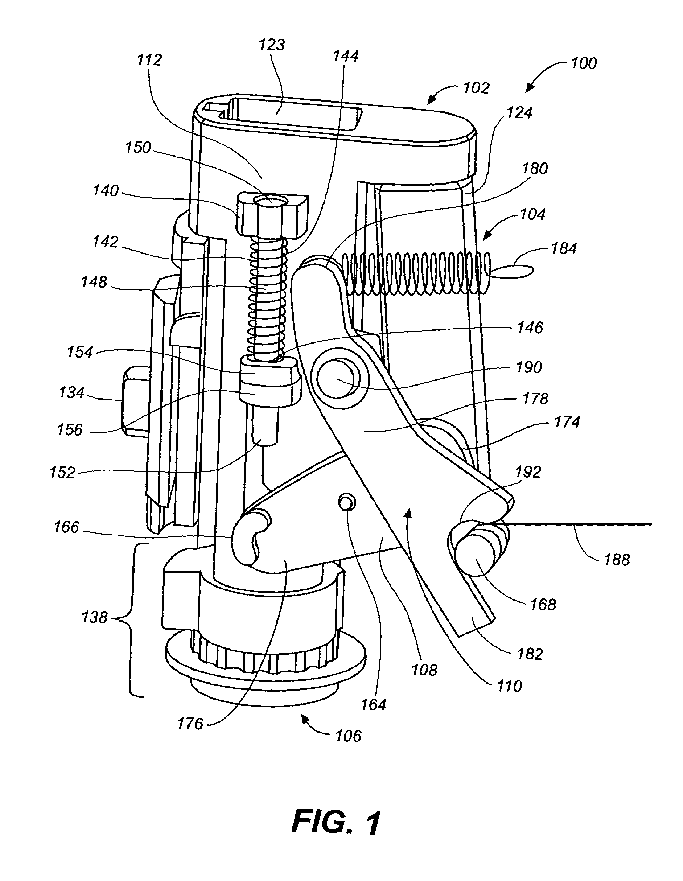

As is described in more detail below with respect to specific embodiments illustrated in the FIGs. 1, lancing apparatuses according to embodiments of the present invention include an inner housing, a firing mechanism, a lancing mechanism and a linkage arm. The firing mechanism is configured for producing a firing force in a first direction. The lancing mechanism is configured for delivering a lancing force in a second direction with the second direction being toward a target site and in opposition to the first direction. The linkage arm is pivotably attached to the housing and has first and second ends engaged to the firing and lancing mechanisms, respectively. During use, pivoting of the linkage arm converts the firing force in the first direction into the lancing force in the opposing second direction.

Lancing apparatus according to embodiments of the present invention are beneficially compact and relatively simple in construction. In addition, post-launching recoil is minimized by the lancing direction being in opposition to the direction of the firing force provided by the firing mechanism, thus acting to reduce pain associated with uncontrolled recoil. In addition, the opposing lancing and firing forces minimize detrimental effects of motion-based linear impulses emanating from the lancing apparatus by transferring such impulses to a housing, rather than to a target site on a user. Furthermore, lancing apparatus according to the present invention can be configured such that momentum in the second direction associated with the lancing force is essentially equal to momentum in the first direction associated with the firing force, thereby also minimizing the detrimental effects of motion-based linear impulses.

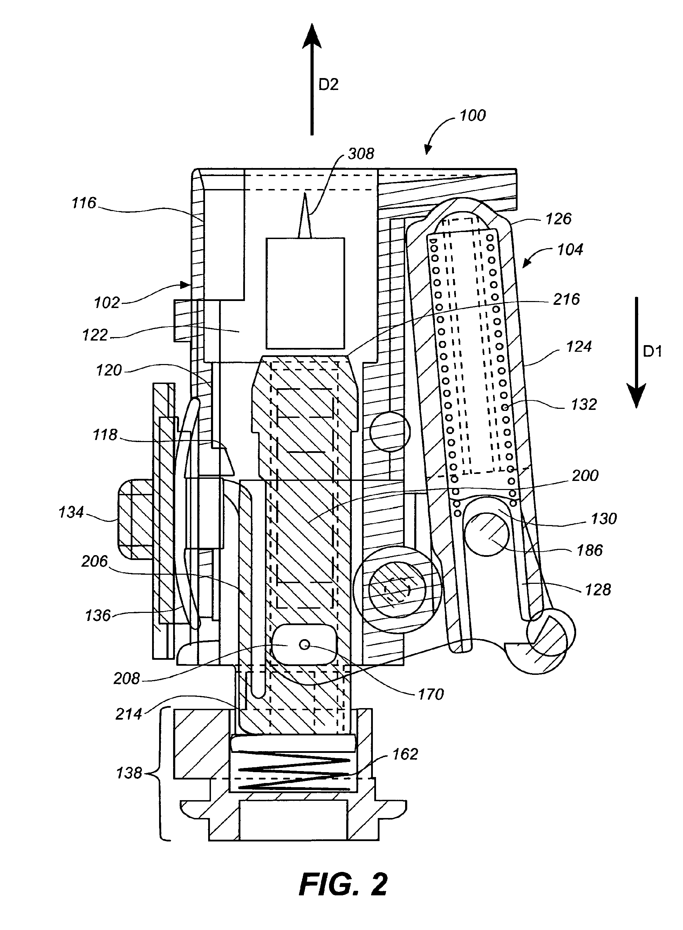

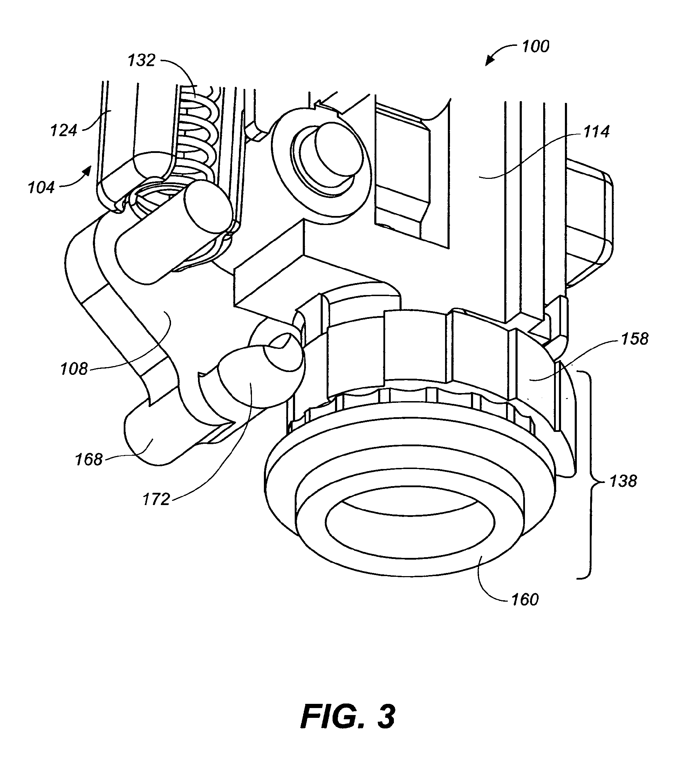

FIG. 1 is a simplified perspective view of a lancing apparatus 100 for lancing a target site (e.g., a dermal tissue target site on a user's fingertip) according to an exemplary embodiment of the present invention. FIG. 2 is a simplified cross-sectional view of lancing apparatus 100 and FIG. 3 is a simplified perspective view of a portion of lancing apparatus 100.

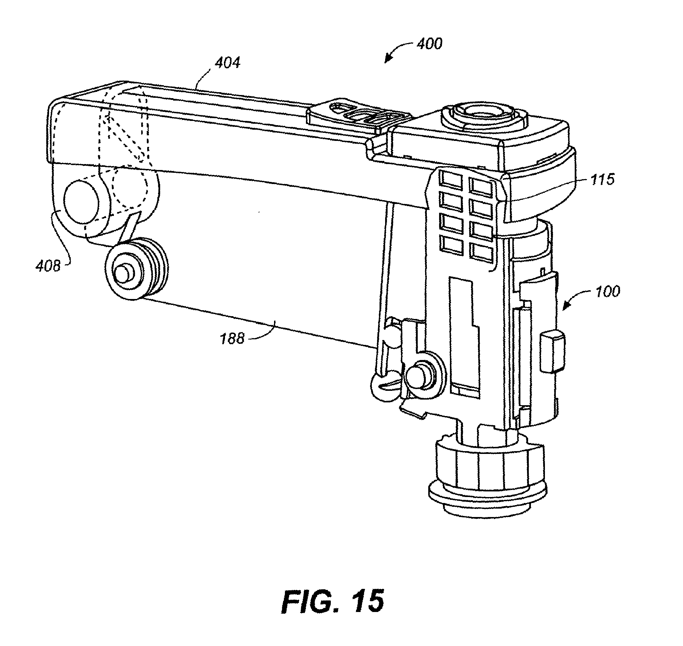

Referring to FIGs. 1, 2 and 3, lancing apparatus 100 includes an inner housing 102, a firing mechanism 104, a lancing mechanism 106, a linkage arm 108 and a priming mechanism 110. Inner housing 102 includes a first outer surface 112, a second outer surface 114 (with windows 115 therethrough, depicted in FIG. 15 only), an inner surface 116, an inner surface protrusion 118, a guide rail 120, a cavity 122 and an opening 123.

Firing mechanism 104 is configured for producing, during use of lancing apparatus 100, a firing force in a first direction as described in more detail below. Firing mechanism 104 includes a casing 124 (with casing distal end 126, casing proximal end 128 and casing cavity 130), a firing spring 132, a trigger button 134 and a trigger spring 136.

Lancing mechanism 106 is configured for delivering a lancing force in a second direction during use of lancing apparatus 100 with the second direction being toward the target site and essentially in opposition to the first direction of the firing force. One skilled in the art will recognize that the firing and lancing forces have associated therewith a firing momentum and a lancing momentum, respectively, due to the mass of moving components of the firing and lancing mechanisms. Moreover, since the second direction is essentially in opposition to the first direction, the lancing momentum is essentially in opposition to the firing momentum. If desired to minimize motion-based linear impulses, the mass of firing and lancing mechanism moving components can be predetermined such that the lancing momentum and firing momentum are essentially equal.

Lancing mechanism 106 includes a lancing depth adjustor 138, a holder 140, a retraction spring 142 (with retraction spring first end 144 and retraction spring second end 146), a rod 148 (with rod first end 150 and rod second end 152), a retraction spring stop 154, and stop 156. In addition, lancing depth adjustor 138 includes a stepped surface 158, a cap 160 and a depth adjustor spring 162.

Linkage arm 108 includes pivot 164 and is pivotably attached to inner housing 102 and by pivot 164. Linkage arm 108 also includes an extension 166, a catch 168, a linkage arm pin 170, a depth adjustor engaging feature 172, a first end 174 and a second end 176. As explained in detail herein, first end 174 is engaged with firing mechanism 104 and second end 176 is engaged with lancing mechanism 106. Moreover, linkage arm 108 is configured to convert a firing force in the first direction (see arrow D1 in FIG. 2) into a lancing force in an essentially opposing second direction (see arrow D2 of FIG. 2). It should be noted that D1 is a direction along the longitudinal axis of firing spring 132 and D2 is along the longitudinal axis of rod 148. Firing spring 132 is attached to first end 174 of linkage arm 108.

Priming mechanism 110 of lancing apparatus 100 includes a priming lever 178 (with priming lever proximal end 180 and priming lever distal end 182), a priming lever spring 184, a priming lever pin 186, a tension member 188, a priming lever pivot 190, and an indent 192.

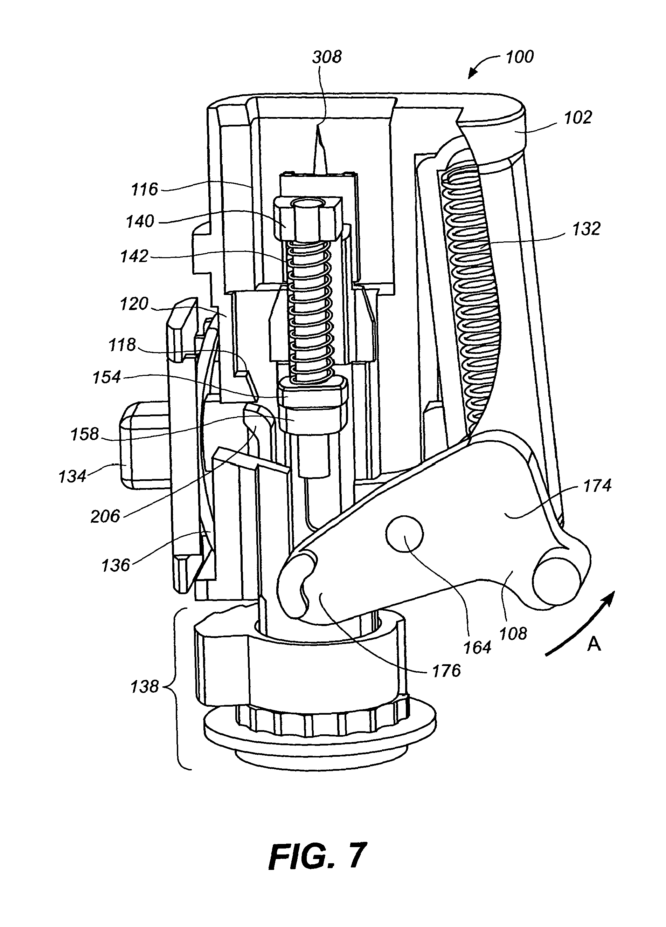

Operation of lancing apparatus 100, as well as the function of inner housing 102, firing mechanism 104, lancing mechanism 106, linkage arm 108, and priming mechanism 110 are explained in detail below, not only with respect to FIGs. 1, 2 and 3, but also with respect to FIGs. 7, 8, 9 and 10.

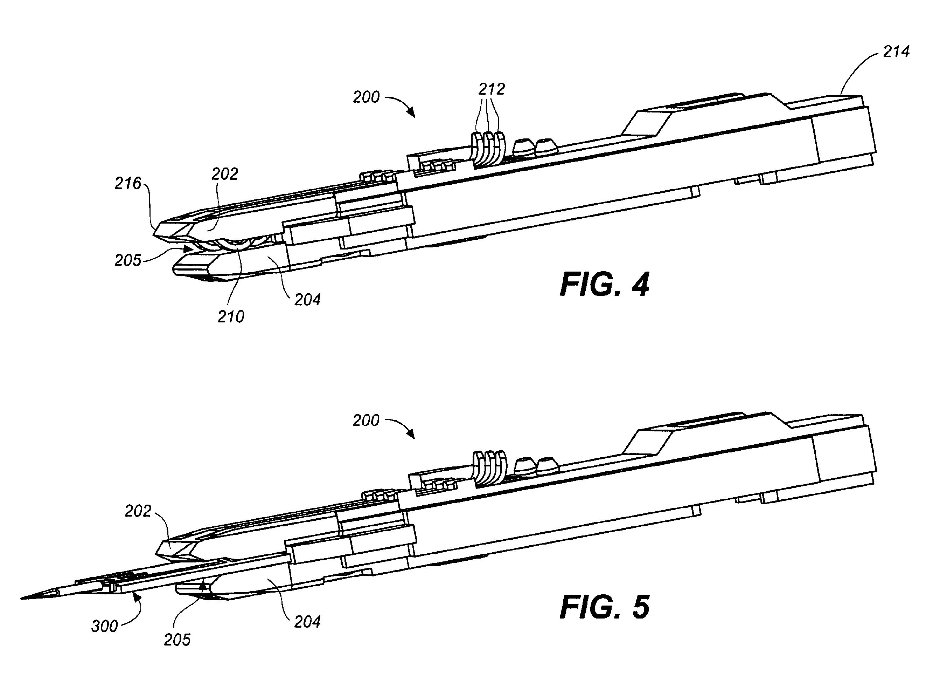

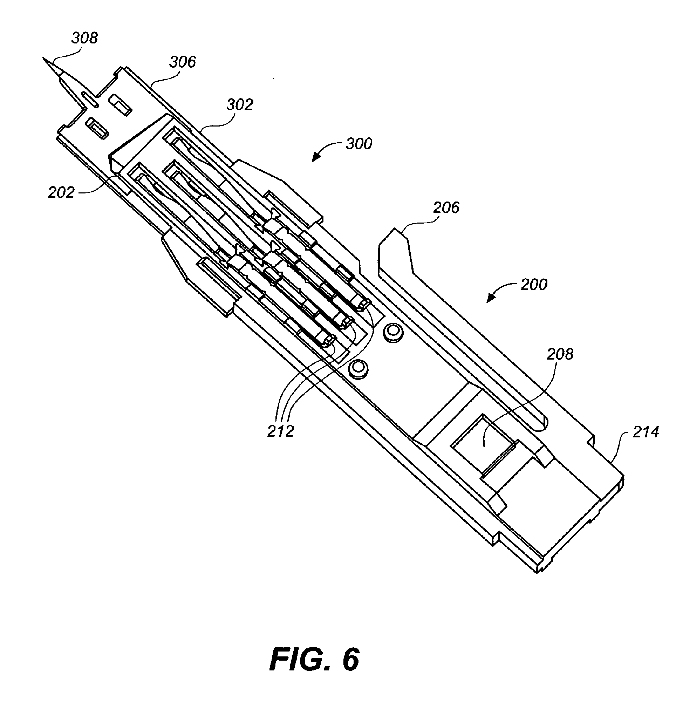

FIG. 2 depicts a connector 200 engaged with lancing apparatus 100 and an integrated medical device 300 engaged with connector 200. Connector 200 and integrated medical device 300 are described below with reference to FIG. 4 (a simplified side view of a connector 200 that can be used with embodiments of lancing apparatuses and analyte monitoring systems according the present invention), FIG. 5 (a simplified side view of the connector of FIG. 4 gripping an integrated medical device 300) and FIG. 6 (a simplified perspective, cut-away view of the connector and integrated medical device of FIG. 5).

Referring to FIGs. 4, 5 and 6, connector 200 includes an upper strip engaging arm 202 and a lower strip engaging arm 204 (with gap 205 therebetween), a connector arm 206, a slot 208, strip engaging elements 210, and electrical lead connections 212. Furthermore, connector 200 has a connector distal end 214 and a connector proximal end 216. Strip engaging elements 210 are in electrical communication with electrical lead connections 212 via a plurality of electrical leads (not shown).

Connector 200 is configured to removably retain (i.e., engage with) an integrated medical device 300 within gap 205 between upper and lower strip engaging arms 204 and 202. Integrated medical device 300 is engaged by strip engaging elements 210, as depicted in FIG. 4.

Furthermore, when connector 200 is operatively engaged with lancing apparatus 100, connector 200 is spring-loaded against depth adjustor spring 162 (see FIG. 2). However, connector 200 can move vertically (in the orientation of FIG. 2) within inner housing 102 when subjected to a lancing force from linkage arm 108. In other words, connector 200 is slideably retained within lancing apparatus 100 while being spring-loaded against depth adjustor spring 162. Connector arm 206 of connector 200 protrudes from connector distal end 214 and engages inner surface protrusion 118 of guide rail 120.

Integrated medical device 300 includes a test strip 302 (with test strip reaction area 304), a dermal tissue penetration member 306 (with lancet 308) and electrical contacts 310. Integrated medical device 300 can be operatively connected to lancing apparatus 100 by connector 200 (see, for example, FIGs. 2 and 5). Lancet 308 is configured to lance dermal tissue of a target site and draw blood into test strip reaction area 304. One skilled in the art will recognize that any suitable integrated medical device can be employed including those described in International Application No. PCT/GB01/0 5634 (published as WO 02/49507 on June 27, 2002) and U.S. Patent Application Publication No. 2003/0143113A2, both of which are fully incorporated herein by reference.

Strip engaging elements 210 and electrical lead connections 212 of connector 200 are configured to provide electrical communication between integrated medical device 300 and an analyte monitoring system (e.g., analyte monitoring system 400 described below). In this regard, strip engaging elements 210 contact test strip 302 of integrated medical device 300 through electrical contacts 310. A further description of connector 200, is included in U.S. Patent Application Publication No. 2005/061700A1.

Lancing apparatus 100 is described herein as employing connector 200 and integrated medical device 300. However, one skilled in the art will recognize that any suitable means can be employed to link a lancing element to lancing apparatus 100 and that lancing apparatuses according to embodiments of the present invention are not limited to use with connector 200 and integrated medical device 300.

Referring again to FIGs. 1, 2 and 3, linkage arm 108 is configured to rotate about pivot 164. As is described in detail below, lancing apparatus 100 is configured in such a way that a firing force in a first direction is converted via pivoting movement of linkage arm 108 into a lancing force in an essentially opposing second direction. Although 180 degrees represents perfect opposition with respect to the first and second directions, an opposition in the range of, for example, +/- 15 degrees about 180 degrees is sufficient to provide the benefits described herein. This lancing force causes lancet 308 of integrated medical device 300 to be launched into a dermal tissue target site.

Priming lever spring 184 connects proximal end 180 of priming lever 178 to an appropriate surface (such as an inner surface of an analyte monitoring system housing (not shown in FIGs. 1-3). Priming lever 178 is adapted to rotate about priming lever pivot 190. Indent 192 of priming lever 178 is configured to retain catch 168 of linkage arm 108. Tension member 188 connects distal end 182 of priming lever 178 to an appropriate related assembly (e.g., to a lid of an analyte monitoring system as described below with respect to FIG. 15) at indent 192.

Priming lever spring 184 can be attached to a suitable related assembly (e.g., an external system housing of an analyte monitoring system as described below). Priming lever spring 184 is employed to place priming lever 178 in a position where priming lever 178 does not interfere with firing mechanism 104 subsequent to the priming of firing mechanism 104.

Once apprised of the present disclosure, one skilled in the art will recognize that priming mechanisms employed in lancing devices according to embodiments of the present invention can take alternative forms to that depicted herein. For example, a suitable priming mechanism can employ a spring-loaded plunger to cooperatively interact with catch 168 rather than the particular lever-based priming mechanism of FIG. 1.

When depressed during use of lancing apparatus 100, trigger button 134 initiates launching of lancet 308 into a target site. Stop 156 is engaged with connector 200 and includes a hole (not shown) through which second end 152 of rod 148 passes. First end 150 of rod 148 is engaged with holder 140 such that rod 148 can slide therethrough. Rod 148 passes through retraction spring 142 and is attached to first outer surface 112 via holder 140.

Second end 146 of retraction spring 142 is retained by spring stop 154 and first end 144 of retraction spring 142 is retained by holder 140. Opening 123 of lancing apparatus 100 is configured to provide for insertion and removal of integrated medical device 300.

Depth adjuster engaging feature 172 of linkage arm 108 is in contact with stepped surface 158 of lancing depth adjuster 138 and serves for a user to set a target site penetration depth of lancet 308. Lancing depth adjuster 138 can be formed of relatively rigid material including, but not limited to, polystyrene, polycarbonate and polyester or any combination thereof.

Trigger spring 136 extends from trigger button 134 to guide rail 120. Linkage arm pin 170 resides within a slot 208 of connector 200 (see, for example, FIG. 2). Casing 124 serves to retain firing spring 132. Moreover, firing spring 132 rests on priming lever pin 186 and resides within casing cavity 130 of casing proximal end 128. Since firing spring 132 is disposed essentially parallel to, and beside, connector 200, lancing apparatus 100 is relatively compact in length.

FIGs. 7, 8, 9 and 10 are simplified perspective cut-away views of lancing apparatus 100 in use, with arrows A, A' and A" indicating movement of a linkage arm of the lancing apparatus.

During use, lancing apparatus 100 is primed by causing priming lever 178 to pivotally rotate about priming lever pivot 190 (see FIG. 1) such that tension is created within tension member 188. As shown in FIG. 7, following priming, linkage arm 108 has been rotated counterclockwise (see arrow A of FIG. 7) about its pivot 164 and has compressed firing spring 132 to a force in the range of, for example, from about 3 Newtons to about 8 Newtons. In addition, retraction spring stop 154 has contacted stop 156 and retraction spring 142 is fully extended between retraction spring stop 154 and holder 140.

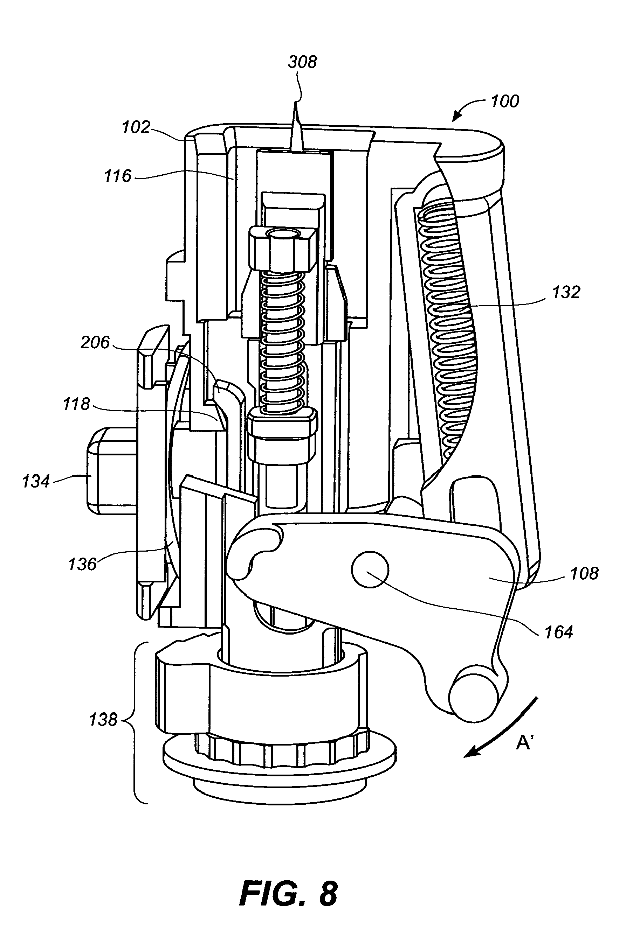

Upon depression of trigger button 134 by a user, arm 206 of connector 200 is displaced away from inner surface protrusion 118. Such displacement of connector arm 206 releases linkage arm 108 to move under the bias of firing spring 132. Firing spring 132 extends and pushes linkage arm 108 clockwise about its pivot 164 (see arrow A' of FIG. 8). As firing spring 132 extends, linkage arm 108 engages slot 208 on connector 200 by means of linkage arm pin 170.

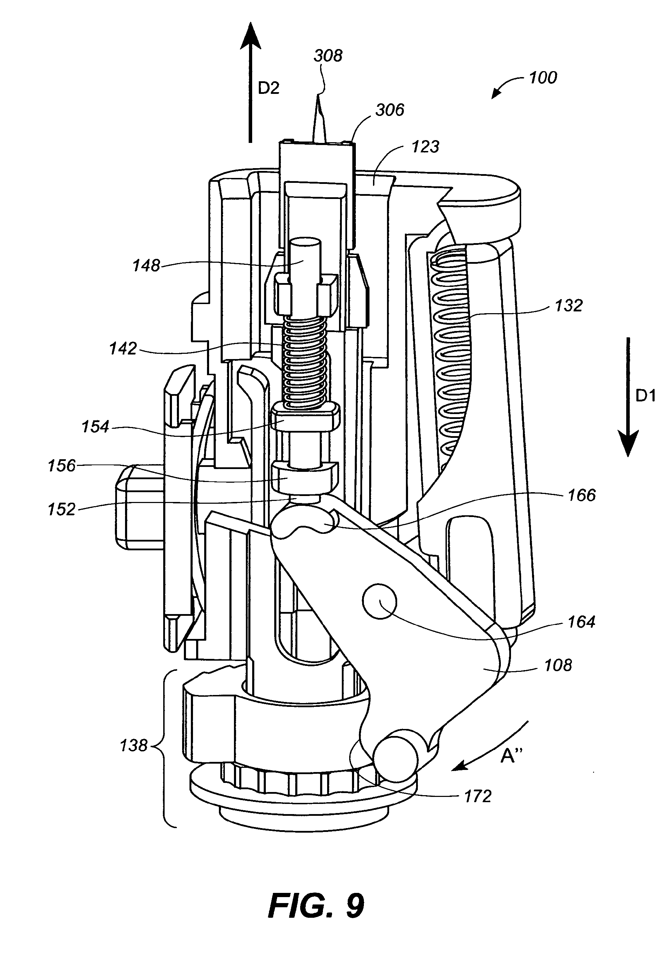

As firing spring 132 continues to extend and exert a firing force on linkage arm 108 (in first direction D1), lancet 308 is extended from lancing apparatus 100 to penetrate a target site (see FIG. 9). This is accomplished as firing spring 132 fully extends causing linkage arm 108 to continue rotating clockwise about pivot 164 (see arrow A" of FIG. 9) such that extension 166 of linkage arm 108 contacts second end 152 of rod 148. This contact and associated momentum impart a lancing force (in second direction D2) that compels rod 148 to move toward opening 123 of lancing apparatus 100, even though there is only a relatively low force (for example, less than about 1.5N) being exerted by extended firing spring 132.

FIG. 9 also depicts the manner in which retraction spring stop 154 has moved away from stop 156 and towards opening 123 of lancing apparatus 100. Movement of rod 148 further compresses retraction spring 142 (which is already compressed to a force, e.g., a force in the range of 2 Newtons to 2.5 Newtons). Although retraction spring 142 may be compressed to a force that is greater than that of the now extended firing spring 132, momentum provides for linkage arm 108 to rotate until it is stopped by contact with stepped surface 158 of lancing depth adjuster 138. This contact prevents further movement of linkage arm 108.

It should be noted that lancing depth adjuster 138 serves to adjust penetration depth by limiting the movement of linkage arm 108. The stepped nature of stepped surface 158 enables a user to determine penetration depth by selecting from a plurality of stepped surface portions (see FIG. 3), each of which is designed to prevent the movement of linkage arm 108 at different rotational points.

The prevention of further linkage arm movement results in the mass associated with connector 200 also stopping, thereby creating an upward impulse. However, the mass of firing spring 132 and the rotation of linkage arm 108 are stopped simultaneously, creating a downward impulse. The upward and downward impulses tend to beneficially balance each other due to the essentially opposing directions of the firing and launching forces.

Since the upward and downward impulses are offset about pivot 164 of linkage arm 108, a rotational impulse is created. However, since connector 200 is guided by guide rail 120, the rotational impulse is not transmitted to the target site but rather is transferred to the inner housing and subsequently to the significant mass of the user's hand. The net effect is that the rotational impulse is not obtrusive and relatively disconcerting to a user.

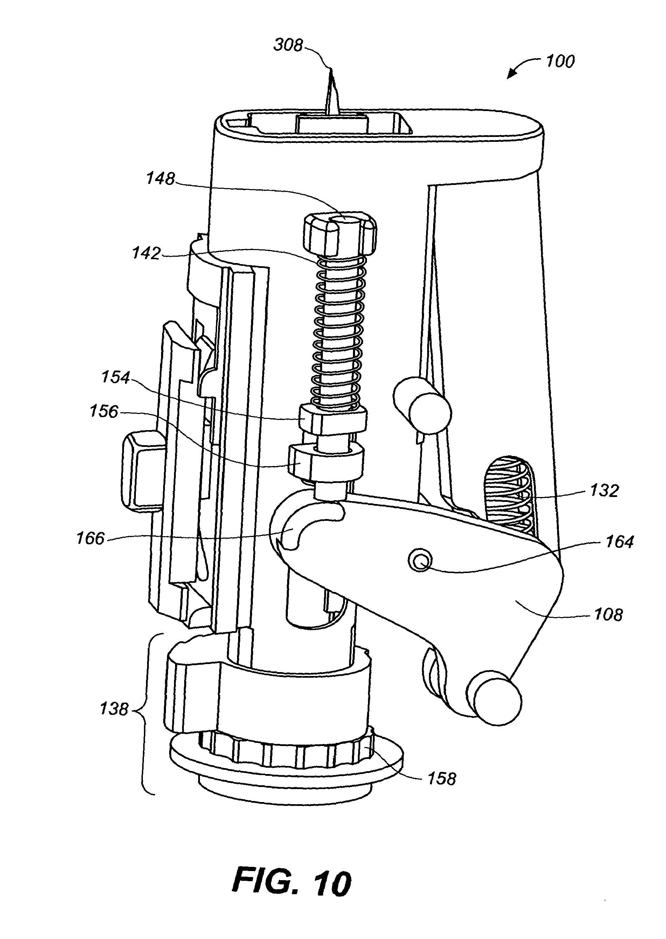

Subsequent to lancing of the target site, the force (e.g., 2 Newtons to 2.5 Newtons) of retraction spring 142 forces serves to force rod 148 to move toward lancing depth adjuster 138 of lancing apparatus 100 while remaining in contact with extension 166 of linkage arm 108 (see FIG. 10). In addition, linkage arm 108 retains connection with connector 200. Rod 148 continues to move in this manner until retraction spring stop 154 contacts stop 156. Linkage arm 108 and connector 200 are thus moved by rod 148 while simultaneously retracting lancet 308 from within the target site to a position, for example, at or slightly below the surface of the target site. A small amount of force remaining in firing spring 132 retains the position of linkage arm 108 and maintains the position of lancet 308 at or slightly below the surface of the target site such that lancet 308 may contact bodily fluid within or expressed from the target site.

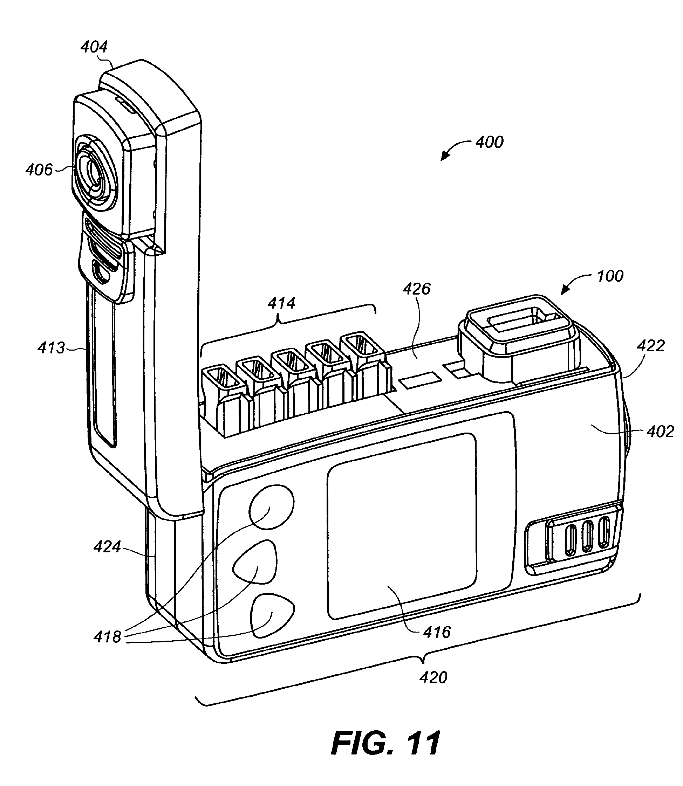

FIG. 11 is a simplified perspective view of an analyte monitoring system 400 according to an exemplary embodiment of the present invention. Analyte monitoring system 400 includes an external system housing 402, a lancing apparatus (i.e., lancing apparatus 100 of, for example, FIG. 1) integrated with external system housing 402, and a meter (not shown) for the determination of an analyte in a bodily fluid sample, the meter at least partially contained with the external system housing.

Analyte monitoring system 400 also includes a lid 404 that is depicted in an open position (i.e., a first position) in FIG. 11. Lid 404 includes a dermal tissue interface 406, a hinge 408 (not shown in FIG. 11, but illustrated in FIG. 15), a lid proximal end 410, a lid distal end 412 and an outer upper surface 413.

Analyte monitoring system 400 also includes a medical device package storage area 414 (depicted in FIG. 11 as storing five medical device packages 500), a visual display 416, and display/control buttons 418. Moreover, external system housing 402 includes a longitudinal side 420, a first end 422, a second end 424, and an inner upper surface 426. Although, for the purpose of explanation only, five medical device packages are depicted in the storage, any suitable number of medical device packages can be stored.

Analyte monitoring systems according to embodiments of the present invention can include any suitable meter including, for example, the electro-chemical based meters described in U.S. Patent No. 6,284,125, U.S. Patent No. 6,413,410 and U.S. Patent Application Publication No. 2003/0143113 A2, each of which is hereby incorporated in full by reference.

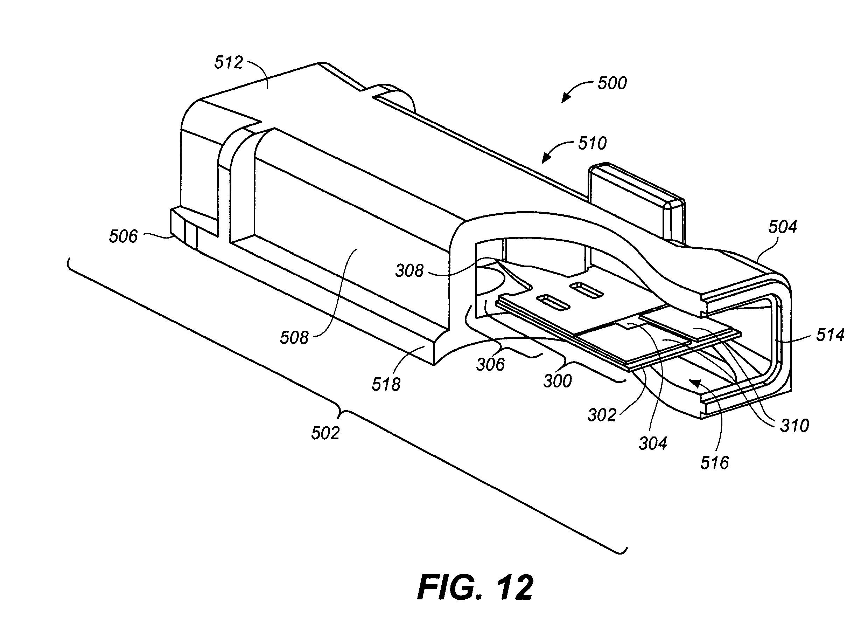

FIG. 12 is a simplified perspective, cut-away view of a medical device package 500 containing an integrated medical device 300 as can be stored in medical device package storage area 414 of analyte monitoring system 400. Medical device package 500 includes a body 502 with a proximal end 504, a distal end 506, a first longitudinal side 508, a second longitudinal side 510, an upper surface 512, a lower surface (not shown in the perspective of FIG. 12), an opening 514, a cavity 516, and one or more wings 518.

Medical device package 500 also includes a foil (not shown) covering opening 514. Opening 514 is located on proximal end 504 and provides access to cavity 516. Cavity 516 is located within body 502 and is configured to securely and removably retain integrated medical device 300.

Wings 518 provide mechanical reference for insertion of medical device package 500 into lancing apparatus 100. Wings 518 extend the length of first and second longitudinal sides 508, 510 of medical device package 500. However, one skilled in the art will recognize that such wings can alternatively extend partially along one or both of longitudinal sides 508, 510, be disposed on upper surface 512 or otherwise disposed on body 502.

Although for descriptive purposes, analyte monitoring system 400 is depicted as storing, and otherwise employing, medical device package 500, any suitable medical device package can be employed with analyte monitoring systems according to embodiments of the present invention. Examples of suitable medical device packages are described in, for example, U.S. Patent Application Publication No. 2005/061700A1.

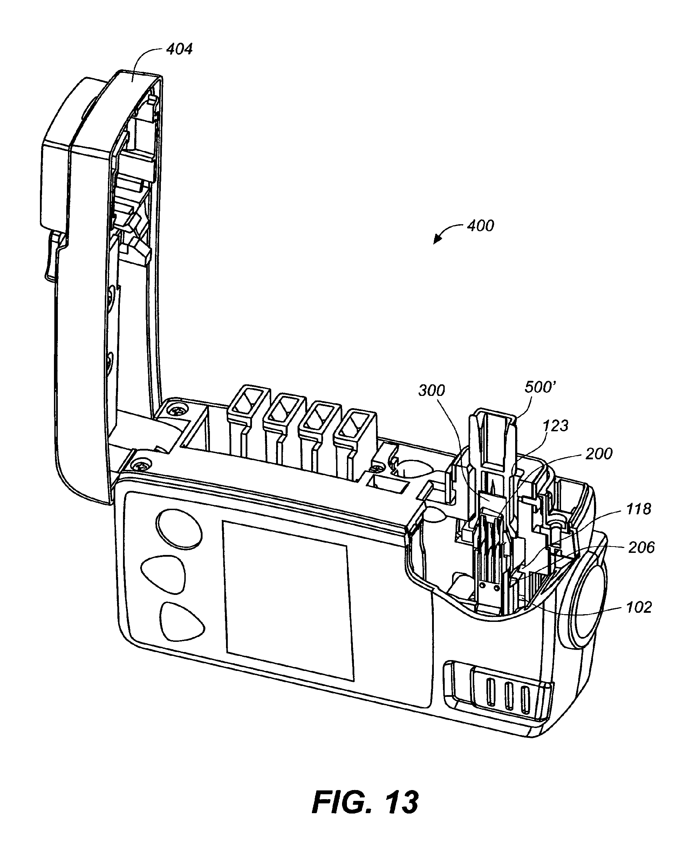

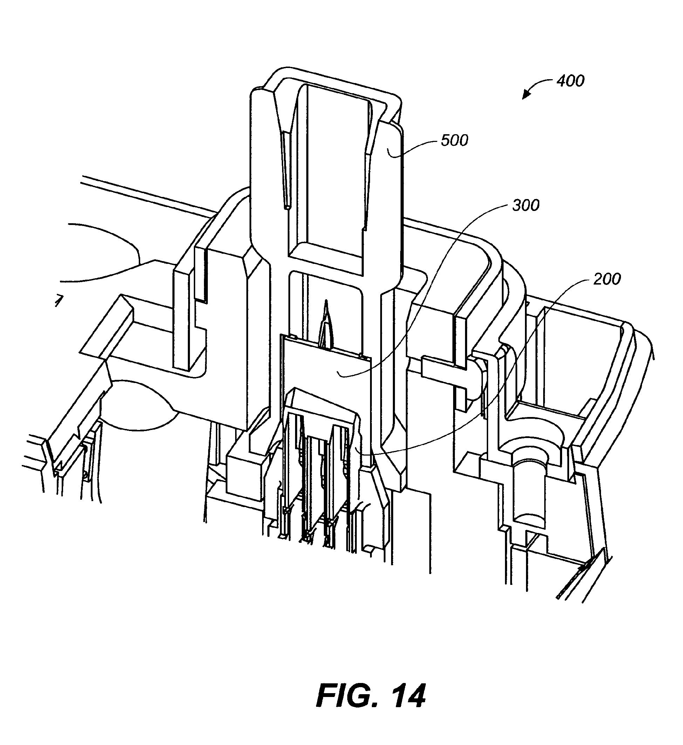

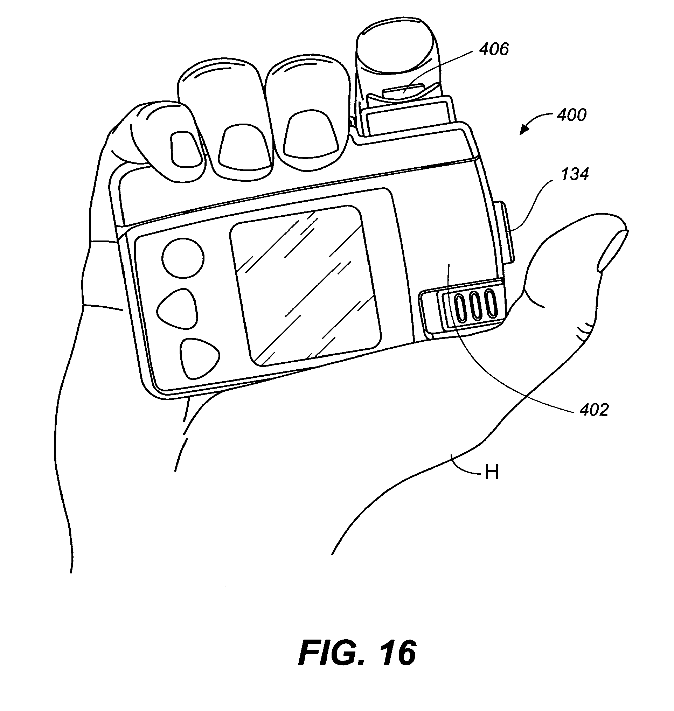

FIG. 13 is a simplified perspective view of analyte monitoring system 400 depicting lid 404 in an open position and a medical device package 500' being employed to insert an integrated medical device into lancing apparatus 100 of the analyte monitoring system. FIG. 14 is a simplified perspective view representing a portion of FIG. 13. FIG. 15 is a simplified perspective view of the lid and lancing apparatus of the monitoring system of FIG. 11 depicting the lid in a closed position (i.e., a second position). FIG. 16 is a simplified perspective view of the analyte monitoring system of FIG. 11 in use in the hand (H) of a user.

Operation of analyte monitoring system 400 is described in detail below with reference to FIGs. 11 through 16. When lid 404 of analyte monitoring system 400 is closed (see, for example, FIG. 15), following the insertion of an integrated medical device 300 into lancing apparatus 100 (see FIG. 14), dermal tissue interface 406 on proximal end 410 of lid 404 is disposed directly over opening 123 of lancing apparatus 100. Therefore, when trigger button 134 is depressed, integrated medical device 300 is launched and lancet 308 penetrates a target site (e.g., a target site on a fingertip of user's hand H) that has been urged against dermal tissue interface 406 (see FIG. 16).

It should be noted that medical device package 500 is removed from opening 123 after integrated medical device 300 has been engaged with connector 200 and before lid 404 is closed.

Visual display 416 is located on first longitudinal side 420 and provides a visual interface to direct a user through the use of analyte monitoring system 400. Display buttons 418 are disposed on longitudinal side 420 near second end 424 and provide for entering commands during use of analyte monitoring system 400.

Lid 404 is disposed above medical device package storage area 414 on outer upper surface 413. Lid 404 can be formed partly, or wholly, of transparent material such that the contents of medical device package storage area 414 can be viewed therethrough. Hinge 408 is located on distal end 412 of lid 404.

Moving lid 404 from a first position (i.e. open) to a second position (i.e. closed) serves to prime lancing apparatus 100 via the operative connection of tension member 188 to hinge 408 (see FIG. 15). Movement of lid 404 to the first position serves to rotate priming lever 178 about priming lever pivot 190 by exercising a tensile force on tension member 188, thus cause priming lever 178 to rotate linkage arm 108 counterclockwise by contacting catch 168 on linkage arm 108.

FIG. 15 depicts windows 115 of lancing apparatus 100. Openings 115 provide for a calibration code or other information on medical device package 500 to be read therethrough. Although, for the purpose of explanation only, eight windows are depicted in the lancing apparatus, any suitable number of windows can be employed.

Referring to FIG. 16 in particular, trigger button 134 of lancing apparatus 100 extends out of first end 422 of external system housing 402 of analyte monitoring system 400. A user can grip and operate analyte monitoring system 400 with a single hand (i.e., hand H of FIG. 16) that includes a finger with a target site. The target site is urged against dermal tissue interface 406 and the user's thumb is employed to depress trigger button 134. Those skilled in the art will recognize that auto-triggering can be employed as an alternative to manual depression of trigger button 134, thus eliminating the need for a trigger button. Such auto-triggering could be initiated, for example, by the target site having been urged against dermal tissue interface 406 with a predetermined force.

Although FIGs. 11 and 13-16 depict a particular embodiment of an analyte monitoring system according to the present invention, one skilled in the art will recognize that analyte monitoring systems for the determination of an analyte (such as glucose) in a bodily fluid sample (e.g., blood) according to the present invention generally include an external system housing, a lancing apparatus and a meter for determination of the analyte. Moreover, the lancing apparatus is integrated with the external system housing and includes an inner housing, a firing mechanism, a lancing mechanism and a linkage arm. The firing mechanism is configured for producing a firing force in a first direction. The lancing mechanism is configured for delivering a lancing force in a second direction with the second direction being toward a target site and in opposition to the first direction. The linkage arm is pivotably attached to the housing and has first and second ends engaged to the firing and lancing mechanisms, respectively. During use, pivoting of the linkage arm converts the firing force in the first direction into the lancing force in the opposing second direction. Such lancing apparatuses are relatively compact and simple to use, requiring only one hand to operate while obtaining a bodily fluid sample from a target site on the same hand.

It should be understood that various alternatives to the embodiments of the invention described herein may be employed in practicing the invention. It is intended that the following claims define the scope of the invention and that structures within the scope of these claims and their equivalents be covered thereby.

## Claims

1. An analyte monitoring system for the determination of an analyte in a bodily fluid sample, the analyte monitoring system comprising:
an external system housing;
a lancing apparatus integrated with the external system housing, the lancing apparatus having:
an inner housing;
a firing mechanism configured for producing, during use, a firing force in a first direction;
a lancing mechanism configured for delivering a lancing force in a second direction during use, the second direction being toward the target site and essentially in opposition to the first direction; and
a linkage arm pivotably attached to the inner housing, the linkage arm including:
a first end engaged with the firing mechanism; and
a second end engaged the lancing mechanism,
wherein the linkage arm is configured to convert the firing force in the first direction into the lancing force in the second direction; and
a meter for the determination of an analyte in a bodily fluid sample, the meter at least partially contained with the external system housing.

2. The analyte monitoring system of claim 1 further including at least one integrated medical device, the integrated medical device including:
a dermal tissue penetration member; and
a test strip.

3. The analyte monitoring system of claim 1 further including a lid and wherein the lancing apparatus includes a priming mechanism and the lid and priming mechanism are operatively connected such that moving the lid to a first position primes the lancing apparatus.

4. The analyte monitoring system of claim 1, wherein the second direction is in opposition to the first direction within a range of +/- 15 degrees about 180 degrees.

5. The analyte monitoring system of claim 4, wherein the second direction is in 180 degrees opposition to the first direction.

6. The analyte monitoring system of claim 1 further including a priming mechanism.

7. The analyte monitoring system of claim 1, wherein the firing mechanism includes a firing spring for producing the firing force.

8. The analyte monitoring system of claim 7, wherein the firing spring has a maximum spring force during use in the range of about 3 Newton to about 8 N.

9. The analyte monitoring system of claim 1, wherein the lancing mechanism includes a retraction spring.

10. The lancing apparatus of claim 1, wherein the meter is configured for determining glucose in a blood sample.

11. The lancing apparatus of claim 1, wherein momentum in the second direction associated with the lancing force is essentially equal to momentum in the first direction associated with the firing force.
